Europäisches Patentamt

European Patent Office

Office européen des brevets

(19)

(11) Veröffentlichungsnummer: **0 241 005**
B1

(12)

# EUROPÄISCHE PATENTSCHRIFT

(45) Veröffentlichungstag der Patentschrift:
31.01.90

(21) Anmeldenummer: 87105185.0

(22) Anmeldetag: 08.04.87

(51) Int. Cl.⁴: **C07C 45/63**, C07C 47/55

(54) Verfahren zur Herstellung von 4-Halogenmethylbenzaldehyd.

(30) Priorität: 08.04.86 DE 3611760

(43) Veröffentlichungstag der Anmeldung:
14.10.87 Patentblatt 87/42

(45) Bekanntmachung des Hinweises auf die Patenterteilung:
31.01.90 Patentblatt 90/5

(84) Benannte Vertragsstaaten:
BE CH DE FR GB IT LI NL

(56) Entgegenhaltungen:
US-A- 4 051 168

THEILHEIMER'S SYNTHETIC METHODS OF ORGANIC
CHEMISTRY, Band 39, Seite 60, 1985, Verlag Karger,
Basel, CH; & A.K. SINHABUBU et al., SYNTHETIC
COMMUNICATIONS, 13, 1983, 677-683
HOUBEN-WEYL "Methoden der Organischen Chemie",
Band V/3, 4. Auflage, Seite 835, 1962, Georg Thieme
Verlag, Stuttgart;
PATENT ABSTRACTS OF JAPAN, Band 3,
Nr. 147 (C-66), 5. Dezember 1979, Seite 99 C 66; & JP - A
- 54 125 628 (BANYU SEIYAKU K.K.) 29.09.1979

(73) Patentinhaber: WACKER-CHEMIE GMBH,
Prinzregentenstrasse 22, D-8000 München 22(DE)

(72) Erfinder: Bräunling, Hermann, Dipl.-Chem.,
Marktlerstrasse 82, D-8263 Burghausen(DE)
Erfinder: Kippe, Dieter, Hechenbergerstrasse 27,
D-8263 Burghausen(DE)

**Beschreibung**

Die Erfindung betrifft die Herstellung von 4-Halogenmethylbenzaldehyd, wobei Halogen für Chlor, Brom oder Jod steht. 4-Halogenmethylbenzaldehyde werden als Ausgangsmaterialien für Pharmazeutica verwendet und dienen als Ausgangsstoffe zur Herstellung von leitfähigen organischen Verbindungen.

Es sind mehrere Verfahren zur Herstellung von 4-Halogenmethylbenzaldehyden bekannt. J. Baker et al. (J. Chem. Soc. 1956, 404) beschreiben die Umsetzung von 4-Chlormethylbenzonitril mit $SnCl_2$ und Chlorwasserstoff zu 4-Chlormethylbenzaldehyd, von 4-Hydroxymethylbenzaldehyd mit $PBr_3$ zu 4-Brommethylbenzaldehyd und von 4-Chlormethylbenzaldehyd mit KJ zu 4-Jodmethylbenzaldehyd. 4-Brommethylbenzaldehyd kann nach J. Syper et al. (Synthesis 1984, 747) durch Oxidation von 4-Brommethylbenzylchlorid mit Selendioxid oder nach der EP-A 45 150 durch Reduktion von 4-Brommethylbenzylchlorid mit Lithium-tri-tert.-butoxyaluminiumhydrid dargestellt werden. Ferner beschreiben G. Drehfahl und G. Plöttner (Chem.Ber. 94, 907 (1961)) eine Synthese, bei der Tolylaldehyd mit rotem Phosphor und Brom zu 4-Brommethylbenzalbromid umgesetzt wird, das man anschließend mit Oxalsäure zu 4-Brommethylbenzaldehyd verseift. Außerdem beschreiben R. Grice und G. Owen (J. Chem. Soc. 1963, 1947) eine Methode, bei der 4-Methoxycarbonylbenzaldimethylacetal mit Lithiumaluminiumhydrid zum 4-Hydroxymethylbenzaldimethylacetal reduziert wird, wonach letzteres durch Behandlung mit gasförmiger HC1 und nachfolgender Umsetzung mit Thionylchlorid in den 4-Chlormethylbenzaldehyd umgewandelt wird.

Gemäß HOUBEN-WEYL, "Methoden der Organischen Chemie", Band V/3, 4. Auflage, S. 835, 1962 ist es weiterhin bekannt, 4-Methoxybenzylchlorid bzw. Tetrakis-Chlormethyldrochinon durch Umsetzung von 4-Methoxybenzylalkohol bzw. Tetrakis-Hydroxymethylhydrochinon mit Chlorwasserstoff herzustellen.

Nachteiligerweise ist keines dieser Verfahren universell anwendbar, d.h. aus einer Ausgangsverbindung durch Zugae von einfachen Basischemikalien das Chlor-, Brom- und Jodderivat zu gewinnen. Weiter werden bei diesen Verfahren Additive verwendet, die entweder als solche hohe Umweltbelastungen und hohe Gefahrenpotentiale hervorrufen wie zum Beispiel $SeO_2$, roter Phosphor, Brom, Chlor, Thionylchlorid oder während der Reaktion zu Nebenprodukten führen wie zum Beispiel Metallsalze, Schwefeldioxid, die aufwendig abgetrennt werden müssen und auch Entsorgungsprobleme mit sich bringen.

Aufgabe der Erfindung war es die Nachteile der bekannten Verfahren zu vermeiden. Gelöst wird die Aufgabe durch ein Verfahren zur Herstellung von 4-Halogenmethylbenzaldehyd, wobei Halogen für Chlor, Brom oder Jod steht, dadurch gekennzeichnet, daß 4-Hydroxymethylbenzaldehyd mit dem entsprechenden gasförmigen Halogenwasserstoff und/oder mit der entsprechenden wäßrigen Halogenwasserstoffsäure umgesetzt wird.

Die Vorteile des erfindungsgemäßen Verfahrens liegen insbesondere darin, daß ausgehend von preiswerten Basischemikalien in guten Ausbeuten 4-Chlor-, 4-Brom- und 4-Jodmethylbenzaldehyd aus einer Ausgangsverbindung in einfachen Reaktionsapparaturen hergestellt werden kann, ohne daß aufwendige Reinigungsschritte nötig sind oder Nebenprodukte anfallen, deren Entsorgung schwierig ist und die Nachteile des Standes der Technik vermieden werden.

Terephthaldialdehyd ist durch katalytische Oxidation von p-Xylol (G. D. Brindelle et al. Ind. Eng. Chem. Prod. Res. Dev. 15(1), 83, 1976) oder durch Dehydrierung von p-Xylylenglycol (US-A 4 239 703) leicht zugänglich.

Die Reduktion des Terephthaldialdehyd zum 4-Hydroxymethylbenzaldehyd kann in jeder geeigneten Weise erfolgen, beispielsweise analog N.M. Weinschenker et al. (J. Org. Chem. 40, 1966, 1975) durch Umsetzung in Toluol mit einem polymeren Organozinnhydridreagenz, wobei n-Butylzinndihydridgruppen an eine Polystyrolmatrix gebunden sind oder analog G.D. Brindell et al. (Ind. Eng. Chem., Prod. Res. Dev. 1, 83, 1976), durch Umsetzung mit Wasserstoff und Palladium auf Aktivkohle in wäßrigem Ethanol, auf die wegen weiterer Einzelheiten im Zusammenhang mit der Reduktion ausdrücklich Bezug genommen wird. Bevorzugt ist die Reduktion nach G.D. Brindell et al. Das durch diese Umsetzung erhaltene Rohprodukt an 4-Hydroxymethylbenzaldehyd, das noch 1 bis 5% Terephthaldialdehyd und 2 bis 10% 1,4-Bishydroxymethylbenzol enthalten kann, wird vorzugsweise ohne weitere Reinigungsschritte verwendet.

Bei der Umsetzung von 4-Hydroxymethylbenzaldehyd wird Halogenwasserstoff erfindungsgemäß gasförmig oder in Wasser gelöst eingesetzt. Bei den wäßrigen Halogenwasserstoffsäuren können verdünnte, beispielsweise 1 n Halogenwasserstoffsäure, bis zu konzentrierte Lösungen, beispielsweise 12 n HC1, 8 n HBr, 7 n HJ, eingesetzt werden. Bevorzugt werden konzentrierte Halogenwasserstoffsäuren verwendet.

Erfindungsgemäß verwendete Halogenwasserstoffe sind Chlorwasserstoff, Bromwasserstoff oder Jodwasserstoff.

Das molare Verhältnis von 4-Hydroxymethylbenzaldehyd zu Halogenwasserstoff, gasförmig oder in wäßriger Lösung ist vorzugsweise 1 : 1 bis 1 : 10, besser 1 : 2 bis 1 : 5, insbesondere 1 : 3 bis 1 : 4.

4-Hydroxymethylbenzaldehyd wird als Schmelze, in wäßrigem Medium oder in einem Lösungsmittel eingesetzt. Verwendete Lösungsmittel sind inerte mit Wasser nicht mischbare Lösungsmittel, beispielsweise chlorierte Kohlenwasserstoffe wie Chloroform oder Methylenchlorid oder beispielsweise Aromaten wie Toluol.

Bei einer bevorzugten Ausführungsform des erfindungsgemäßen Verfahrens wird gasförmiger Halogenwasserstoff innerhalb von vorzugsweise 1 bis 10 Stunden, insbesondere von 2 bis 5 Stunden durch eine Schmelze von 4-Hydroxymethylbenzaldehyd oder durch eine mit Wasser nicht mischbare Lösung von 4-Hydroxymethylbenzaldehyd geleitet. Vorzugsweise wird hierbei der Halogenwasserstoff im Kreis geführt, wobei mitgeführtes Reaktionswasser nach bekannten Methoden z.B.: durch Auskondensieren über eine Zwischenkühlung des Kreisgases, Adsorption an ein Trockenmittel z.B. Kieselgel oder durch Auswaschen mit konzentrierter Schwefelsäure oder Phosphorpentoxid abgetrennt wird, bevor der Halogenwasserstoff wieder der Reaktion zugeführt wird. Die Reaktionstemperatur beim Einleiten in die Schmelze beträgt vorzugsweise 80 bis 200°C, besser 100 bis 160°C, insbesondere 120 bis 140°C. Die Reaktionstemperatur beim Einleiten in eine Lösung beträgt vorzugsweise 0 bis 100°C, insbesondere 40 bis 80°C.

Das entstehende Reaktionswasser wird mit dem Halogenwasserstoff mitgeführt beziehungweise aus dem mit Wasser nicht mischbare Lösungsmittel zusammen mit unverbrauchtem Halogenwasserstoff als konzentrierte Halogenwasserstoffsäure ausgeschieden.

Bei einer weiteren bevorzugten Ausführungsform des erfindungsgemäßen Verfahrens wird 4-Hydroxymethylbenzaldehyd ggf. unter Zugabe eines mit Wasser nicht mischbaren Lösungsmittels mit wäßriger Halogenwasserstoffsäure umgesetzt. Falls nötig kann hierbei der Halogenwasserstoffgehalt durch Einleiten von zusätzlichem gasförmigem Halogenwasserstoff erhöht werden. Die Reaktionstemperatur beträgt hierbei vorzugsweise 0 bis 100°C, insbesondere 40 bis 80°C. Die Reaktionszeit ist vorzugsweise 1 bis 24 Stunden, insbesondere 2 bis 6 Stunden. Bei der Umsetzung mit wäßriger Halogenwasserstoffsäure ohne Zugabe eines Lösungsmittels kristallisiert das Produkt direkt aus. Bei der Umsetzung mit wäßriger Halogenwasserstoffsäure unter Zugabe eines mit Wasser nicht mischbaren Lösungsmittels wird das Produkt durch das Lösungsmittel aus der wäßrigen Phase extrahiert. Hierbei wird vorzugsweise das Reaktionsprodukt in der dem Fachmann bekannten Weise im Gegenstromverfahren extrahiert. Die nach Reaktion anfallende verdünnte Halogenwasserstoffsäure kann durch bekannte Maßnahmen, z.B. durch Destillation wieder aufkonzentriert werden.

Die Erfindung wird nun anhand von Beispielen näher erläutert.

### Beispiel 1:

20 g Terephthaldialdehyd (0,149 mol) und 100 mg Pd/C (10% Pd auf Aktivkohle) wurden in 100 ml 80 proz. Ethanol unter Argon in einem 500 ml Dreihalskolben aufgeschlämmt. Der Kolben war mit gasdichtem Rührverschluß und Blattrührer versehen. Nachdem das Argon durch Wasserstoff verdrängt worden war, wurde bis zur Aufnahme der theoretischen Menge Wasserstoff (3,34 l) kräftig gerührt. Nach Evakuieren zur Entfernung des Wasserstoffes wurde der Katalysator abgesaugt. Aus der am Rotationsverdampfer bei 60° C eingedampften Lösung wurden 20,5 g Rohprodukt erhalten, das 90 mol% 4-Hydroxymethylbenzaldehyd, 5 mol% unumgesetzten Terephthaldialdehyd und 5 mol% 1,4-Bishydroxymethylbenzol enthielt.

### Beispiel 2:

In einem 250 ml Dreihalskolben mit Magnetrührer und einem bis zum Boden reichenden Gaseinleitungsrohr wurden 94,4 g 4-Hydroxymethylbenzaldehyd (90 proz., Verunreinigungen 1,4-Bis-hydroxymethylbenzol und Terephthaldialdehyd) vorgelegt. In die aufgeschmolzene Masse wurde HCl-Gas eingeleitet, wobei sich die Temperatur auf 75°C erhöhte. Nach 2-stündigem Durchleiten von HCl wurde das Gemisch auf 140°C im Ölbad erwärmt und weitere 2½ Stunden HCl durchgeleitet. Destillation bei 17 Torr (Kp 136°C) ergab 88,6 g eines weissen kristallinen Produktes, das nach NMR 87,5 Gew.-% 4-Chlormethylbenzaldehyd, 10,2 Gew.-% 1,4-Bischlormethylbenzol, 2,3 Gew.-% Terephthaldialdehyd enthielt. Als Rückstand verblieben 14,5 g einer zähen, nicht flüchtigen Masse, in die erneut 15 Stunden bei Raumtemperatur und dann 1,5 Stunden bei 150°C HCl eingeleitet wurde.

Danach konnten daraus wieder 6,5 g 95% 4-Chlormethylbenzaldehyd abdestilliert werden. Gesamtausbeute, berechnet auf reinem Aldehyd = 83,7 g = 86,8% d.Th. bezogen auf den Reinaldehydgehalt des Ausgangsproduktes. HNMR; 4,64 s 2H; 7,57 d 2H; 7,89 d 2H, J = 8 Hz; 10,03 s 1H.

### Beispiel 3:

20 g (0,132 Mol) 90 proz. 4-Hydroxymethylbenzaldehyd, Verunreinigung 1% Terephthaldialdehyd, 9% 1,4 Bis-hydroxymethylbenzol wurden bei Raumtemperatur mit 33 ml 48 proz. Bromwasserstoffsäure (0,29 Mol) versetzt und danach 2 Stunden bei 65°C gerührt. Nach 18 Stunden bei 4°C im Kühlschrank wurde der Kristallbrei abgesaugt, mit 5 ml 48 proz. HBr gewaschen und im Exsiccator über Phosphorpentoxyd getrocknet. Rohausbeute 16,8 g. Gehalt nach NMR 85,9 Gew.-% = 54% d. Th. 4-Brommethylbenzaldehyd HNMR: 4,51 2H; 7,55 d 2H; 7,85 d 2H, J = 8 Hz; 10,01 s 1H.

### Beispiel 4:

20 g (0,132 Mol) 4-Hydroxymethylbenzaldehyd (90 proz.) wurden mit 66 g 57 proz. Jodwasserstoffsäure (0,29 Mol) versetzt, 2 Stunden unter Argon bei 80°C gehalten, wobei das Produkt bereits auskristallisierte und der Ansatz zu einem festen Brei erstarrte. Nach weiterem 16-stündigem Stehen bei 4°C wurde die überschüssige Jodwasserstoffsäure abgesaugt und das Produkt über Phosphorpentoxyd getrocknet. Das noch etwas feuchte Rohprodukt (42,5 g) wurde in einer Sublimationsapparatur zunächst bei 0,005 Torr und 60°C von Feuchtigkeit und Jodverunreinigungen befreit. Danach bei 95°C durch Sublimation gereinigt. Man erhielt 26,14 g Sublimat (durch geringen Jodgehalt noch etwas braungefärbt) das 83 Gew.-% 4-Jodmethylbenzaldehyd enthielt. Ausbeute berechnet auf reinem Aldehyd 21,7 g = 66% d. Th. Als Sublimationsrückstand verblieben 5,2 g eines schmutziggrünen Pulvers, das nicht mehr in Chloroform löslich war. Bei der 2. Sublimation blieben rund 350 mg Rückstand. Es wurden 20,81 g Sublimat mit 88% 4-Jodmethylbenzaldehyd erhalten verunreinigt mit 1,4-Bisjodmethylbenzol und Terephthaldialdehyd erhalten. Zweimaliges Umkristallisieren aus je 400 ml Cyclohexan lieferte 13 g 4-Jodmethylbenzaldehyd Reinheit: 99% HNMR: 4,41 s 1H; 7,45 d 2H; 7,68 d 2H, J = 8 Hz; 9,91 s 1H.

Beispiel 5:

20 g (0,1332 Mol) 90% 4-Hydroxymethylbenzaldehyd mit 50 ml 37 proz. HCl (0,58 Mol) und 200 ml Toluol wurden 2 Stunden bei 80°C unter Argonatmosphäre gerührt. Nach Abkühlen auf Raumtemperatur wurde die wäßrige Phase abgetrennt und die Toluolphase in Vakuum eingedampft. Man erhielt 16,2 g des Rohprodukts, das nach NMR 78,8 Gew.-% 4-Chlormethylbenzaldehyd, 10 Gew.-% 1,4 Bischlormethylbenzol und 11,2 Gew.-% einer unbekannten Substanz enthielt. Aus dem NMR errechnete sich eine Ausbeute an 4-Chlormethylbenzaldehyd von 12,8 g = 62,5% d. Th.

Beispiel 6:

40 g (0,264 Mol) 90 proz. 4-Hydroxymethylbenzaldehyd wurde mit 132 ml 48 proz. HBr (1,17 Mol) und 250 ml Toluol 2 Stunden bei 80°C gerührt. Die wäßrige Schicht wurde noch warm abgetrennt, worauf die Toluollösung 18 Stunden bei 4°C stehengelassen wurde. Die dabei ausgefallenen Kristalle wurden abgesaugt, mit wenig Toluol nachgewaschen und bei 40°C mit 0,1 Torr getrocknet. Ausbeute 36,85 g 90 proz. 4-Brommethylbenzaldehyd verunreinigt mit 1,4 Bisbrommethylbenzol. Aus der Mutterlauge erhielt man nach Abdampfen des Lösungsmittels und Trocknen im Wasserstrahlvakuum bei 40°C und 0,1 Torr weitere 20 g, die laut NMR 68% 4-Brommethylbenzaldehyd enthielten. Gesamtausbeute bezogen auf reinen 4-Brommethylbenzaldehyd = 46,4 g = 88% d. Th.

Beispiel 7:

Gemäß Beispiel 6 wurden 17,13 g 84 proz. 4-Hydroxymethylbenzaldehyd (0,106 mol) (Restverunreinigung: 10% 1,4-Bishydroxymethylbenzol + 5% Terephthaldialdehyd) mit 38,8 ml 57 proz. Jodwasserstoffsäure (0,294 mol) umgesetzt. Aus der organischen Schicht wurde nach dem Stehen bei 4°C 24,7 g Rohprodukt erhalten, die ohne weitere Trocknung sofort bei 90°C und 0,01 Torr sublimiert wurden. Man erhielt 21,95 g Sublimat, das nach NMR 88,8 mol% 4-Jodmethylbenzaldehyd enthielt. Durch Eindampfen der organischen Mutterlauge im Wasserstrahlvakuum und Trocknen bei 50°C und 0,1 Torr wurde eine zweite Fraktion mit 68,5 mol% 4-Jodmethylbenzaldehyd erhalten. Daraus berechnet sich eine Gesamtausbeute an 4-Jodmethylbenzaldehyd von 23,6 g = 89% d. Th.

**Patentansprüche**

1. Verfahren zur Herstellung von 4-Halogenmethylbenzaldehyd, wobei Halogen für Chlor, Brom oder Jod steht, dadurch gekennzeichnet, daß 4-Hydroxymethylbenzaldehyd mit dem entsprechenden gasförmigen Halogenwasserstoff und/oder mit der entsprechenden wäßrigen Halogenwasserstoffsäure umgesetzt wird.

2. Verfahren nach Anspruch 1, dadurch gekennzeichnet, daß ein mit Wasser nicht mischbares Lösungsmittel zugegeben wird.

3. Verfahren nach Anspruch 1, dadurch gekennzeichnet, daß 4-Hydroxymethylbenzaldehyd als Schmelze mit gasförmigem Halogenwasserstoff umgesetzt wird.

**Revendications**

1. Procédé de préparation d'un halogénométhyl-4 benzaldéhyde dans lequel l'halogène est le chlore, le brome ou l'iode, procédé caractérisé en ce qu'on fait réagir l'hydroxyméthyl-4 benzaldéhyde avec l'halogénure d'hydrogène gazeux correspondant et/ou avec l'acide halogénhydrique aqueux correspondant.

2. Procédé selon la revendication 1 caractérisé en ce qu'on ajoute un solvant non miscible à l'eau.

3. Procédé selon la revendication 1 caractérisé en ce qu'on fait réagir l'hydroxyméthyl-4 benzaldéhyde sous la forme d'un produit fondu avec l'halogénure d'hydrogène gazeux.

**Claims**

1. Process for the preparation of 4-halogenomethylbenzaldehyde in which halogeno stands for chloro, bromo or iodo, characterized in that 4-hydroxymethylbenzaldehyde is reacted with the corresponding gaseous hydrogen halide and/or with the corresponding aqueous hydrohalic acid.

2. Process according to Claim 1, characterized in that a water-immiscible solvent is added.

3. Process according to Claim 1, characterized in that 4-hydroxymethylbenzaldehyde is reacted as a melt with gaseous hydrogen halide.